# EUROPEAN PATENT APPLICATION

(11) **EP 2 666 771 A1**
(43) Date of publication of application: **27.11.2013**
(21) Application number: 12169182.8
(22) Date of filing: 24.05.2012
(51) Int. Cl.: C07D 317/26

(54) **Synthesis of Aminocyclopentanetriol Derivatives**

(71) Applicant: LEK Pharmaceuticals d.d., 1526 Ljubljana (SI)
(72) Inventor: Sterk, Damjan, 1526 Ljubliana (SI); Casar Zdenko, 1526 Ljublijana (SI)
(74) Representative: Kunic Tesovic, Barbara

(57) **Abstract**

The present invention relates to the field of organic synthesis and describes the synthesis of specific intermediates suitable for the preparation of triazolopyrimidine compounds such as ticagrelor.
The present invention provides in particular a process for the preparation of a compound of formula V 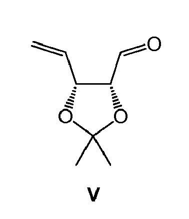 comprising:
a) providing a compound of formula IV 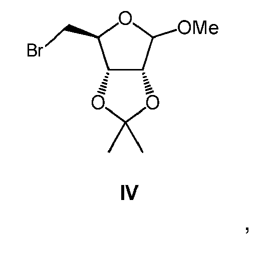 , and
b) reducing the compound of formula IV with activated zinc in the presence of copper to give the compound of formula V.

## Description

The present invention relates to the field of organic synthesis, in particular to the synthesis of specific aminocyclopentanetriol derivatives as intermediates for preparation of P2Y12 receptor antagonists.

### Background of invention

Aminocyclopentanetriol moiety is an essential part of a novel pharmaceutical ingredient characterised by generic name ticagrelor and marketed as Brilique^{®}, Possia^{®} or Brilinta^{®}. Chemically it is 3-[7-[[(1*R*,2*S*)-2-(3,4-difluorophenyl)cyclopropyl]amino]-5-(propylthio)-3*H-*1,2,3-triazolo[4,5-d] pyrimidin-3-yl]-5-(2-hydroxyethoxy)-(1*S*,2*S*,3*R*,5*S*)-1,2-cyclopentanediol) having the following structural formula.

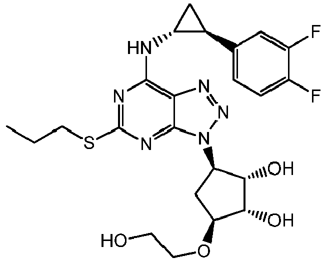

Ticagrelor shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 00/34283).

In general, aminocyclopentanetriol building blocks of formula **A,** wherein PG₁, PG₂, and R₁ are selected from hydrogen or corresponding protecting groups and R is selected from hydrogen, hydroxyethyl or protected hydroxyethyl are various intermediates for the synthesis of ticagrelor with **IX, XIV,** and XV being the key representatives (Scheme 1).

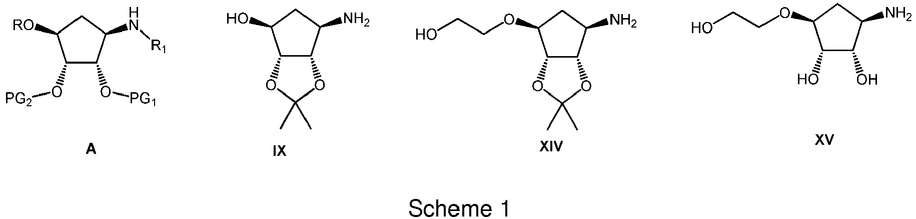

While **XIV** is described in special publications of ticagrelor and similar anticoagulants and **XV** has even not been described, the description of preparation of **IX** is ready available in various scientific publication. An overview of synthetic routes to **IX** for specific use in ticagrelor synthesis is described in Chinese Journal of Pharmaceuticals 2011, 42, 146-150, wherein four main approaches are described:
A) Racemic analogue of **IX** can be prepared by addition of nitroso compounds such as (3,5-dinitrophenyl)(nitroso)methanone (Scheme 2; also Can. J. Chem. 1976, 54, 867-870; Helv. Chem. Acta, 1983, 66, 1915-1921), 1-chloro-1-nitrosocyclohexane (Scheme 3; also Tetrahedron, 1997, 53, 3347-3362) to cyclopentadiene. In these cases the need for separation of isomers is required, which makes these approaches less efficient. Route to **IX** via chiral nitroso compounds such as (R)-tert-butyl (1-nitroso-1-oxopropan-2-yl)carbamate (Scheme 4; also Tetrahedron, 1996, 52, 291-304; Synlett, 2007, 16, 2541-2544; Tetrahedron Lett., 2000, 41, 9537-9540) is also described. All of these approaches start from cyclopentadiene which needs to be prepared directly before use and from nitroso compound which are not easily prepared. These approaches also depend on the use of heavy metals such as osmium or manganese for dihydroxylation. 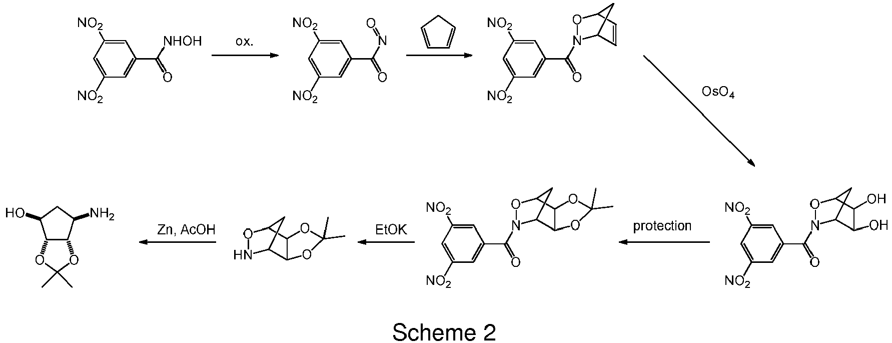 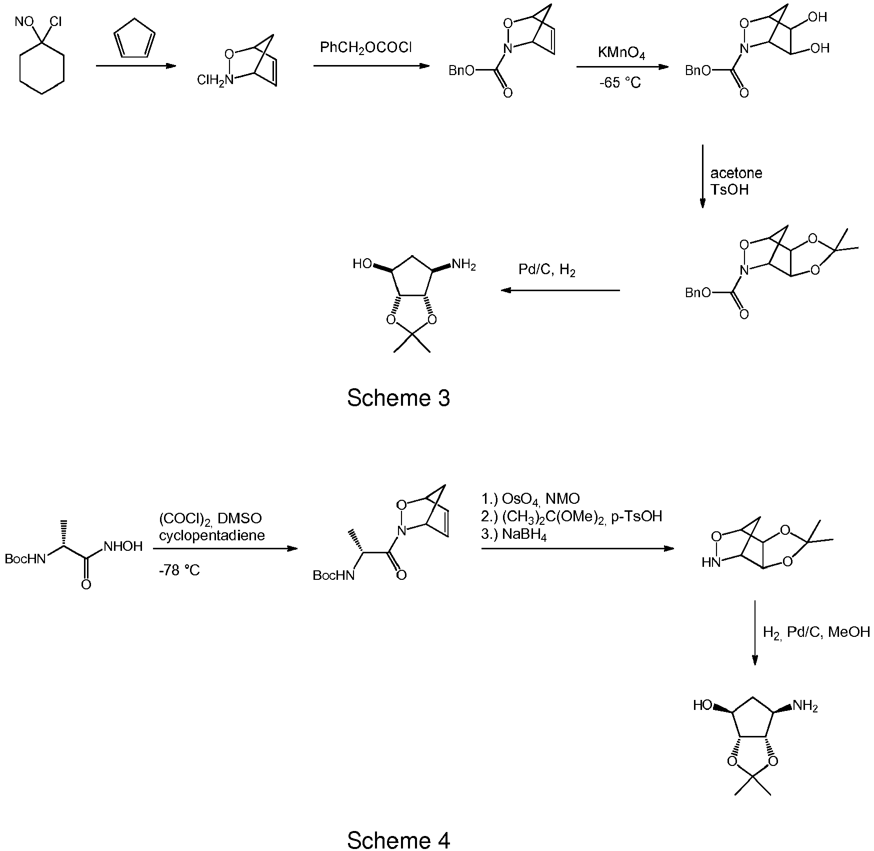
B) In another approach, described in Bioorg. Med. Chem. Lett. 2007, 6013-6018, **IX** is prepared from chiral (1R,4S)-4-hydroxycyclopent-2-en-1-yl acetate via a four steps sequence. Use of expensive chiral starting materials, expensive reagents ((Boc)₂NNa, Pd(PPh₃)₄) and toxic osmium tetroxide makes process unsuitable for industry (Scheme 5). 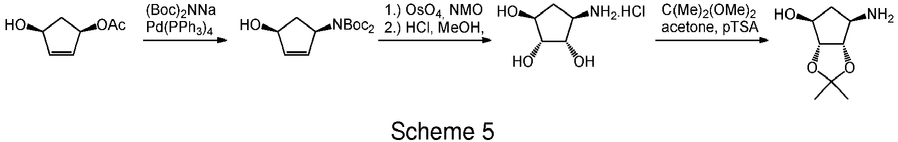
C) Yet another approach to **IX** (Scheme 6; also Tetrahedron, 1993, 49, 6669-6694; Tetrahedron: Asymmetry, 1991, 2, 961-964; Tetrahedron: Asymmetry, 1997, 8, 2249-2256), is a 7 step synthesis starting from ribonolactone incorporating the use of hazardous dibutylaluminium hydride (DIBALH), diethyl azodicarboxylate (DEAD), lithium aluminum hydride, AIBN or toxic (tributyltin) reagents. 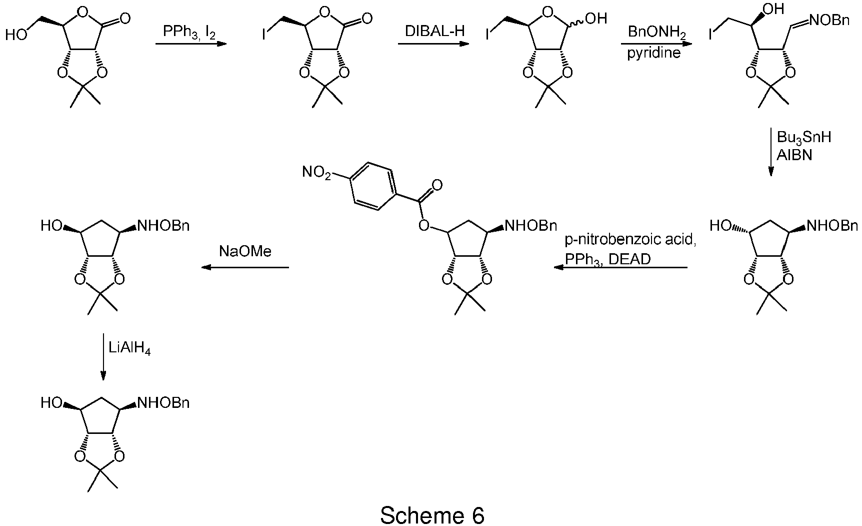
D) Yet another synthesis described in Chinese Journal of Pharmaceuticals 2011, 42, 146-150 starts from a ribose derivative, but eliminates major hazardous reagents in the synthesis of item C) (Scheme 7).

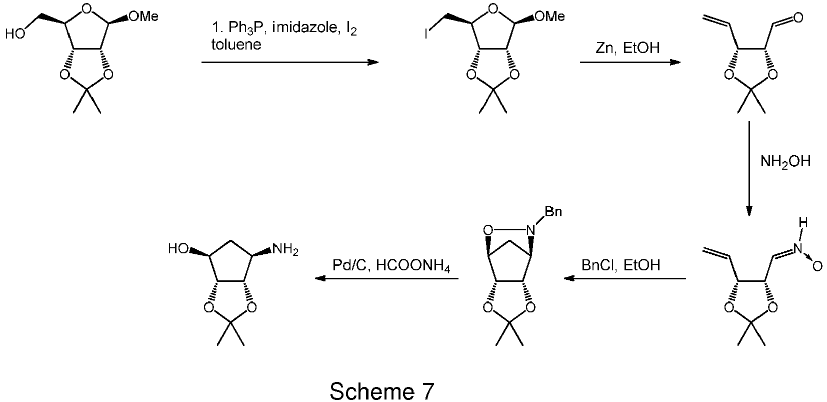

While the process for preparation of **IX** according to item D) has the characteristics of being industrially applicable, it still has some drawbacks. Use of triphenylphosphine, for instance, generates triphenylphosphine oxide, which is very difficult for elimination from the process, especially in the process in which all intermediates are in the form of oil. Such process needs column chromatography at least in one step in order to obtain an intermediate suitable for preparation of pharmaceutical ingredients.

According to WO 00/34283, **XIV,** a higher intermediate for the synthesis of ticagrelor, is prepared from **IX** by protection of the amino group, followed by condensation of bromoacetate, reduction and final deprotection (Scheme 8).

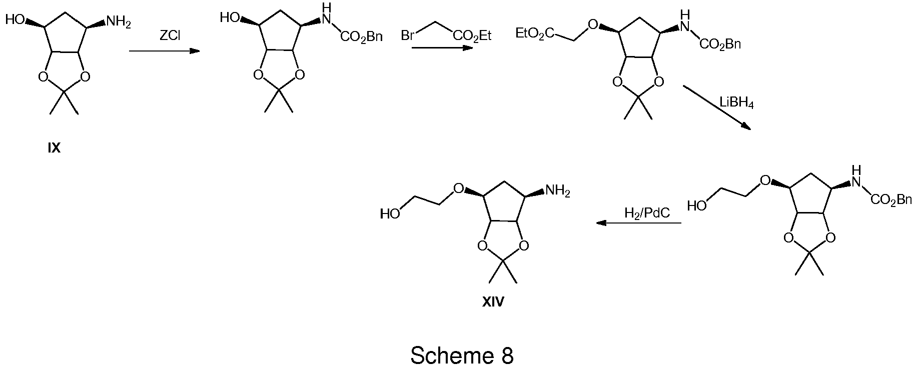

As becomes apparent from the above, there is still need for industrially applicable synthesis of aminocyclopentanetriol derivatives as intermediates for the synthesis of ticagrelor.

### Summary of the Invention

The object of the present invention was to provide an industrially applicable and economically improved process of preparation of aminocyclopentatriol derivatives as intermediates in the synthesis of ticagrelor.

The present invention provides a process for the preparation of a compound of formula V 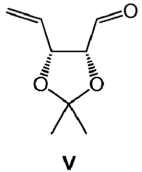 comprising:
a) providing a compound of formula **IV** 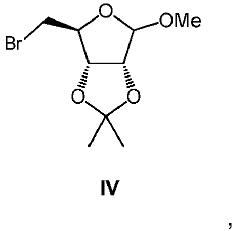 and
b) reducing the compound of formula I**V** with activated zinc in the presence of copper to give the compound of formula **V**.

The process defined above in the reduction step b) utilizes catalytic amounts of copper salts and activated zinc or activated zinc-copper couple, which allows for preparation of the compound of formula V starting from iodine free intermediate. In addition, said process can be conducted at temperatures between 20 and 40°C, which eliminates the need for conducting the reaction at cryogenic or reflux temperatures. Both elimination of cryogenic or reflux temperatures and replacement of iodine for cheaper bromine as well as omission of certain reagent such as triphenylphosphine make the process industrially friendlier and more competitive.

### Description of the Invention

Aspects, advantageous features and preferred embodiments of the present invention will be described in further detail below, noting however that such aspects, advantages features as well as embodiments and examples are presented for illustrative purposes only and shall not limit the invention in any way.

An embodiment of the present invention is to provide industrially applicable and economical process for obtaining aminocyclopentanetriol derivatives of formula **A** 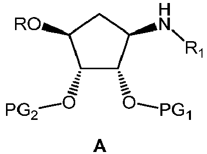 wherein PG₁, PG₂, and R₁ are selected from hydrogen or corresponding protecting groups and R is selected from hydrogen, hydroxyethyl or protected hydroxyethyl.

In case PG₁ and PG₂ are protecting groups they belong to a common alkylene group, preferably to a substituted methylene group, most preferably to the isopropylidene group.

R₁ can be any suitable amino protecting group known to a skilled person, for example such a group can be selected from the group consisting of *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), fluorenylmethoxycarbonyl (Fmoc), C₄-C₅-*tert*-alkyl, for example *tert-*butyl (*t*-Bu), or mono, di- or triphenyl substituted methyl, for example benzyl (Bn). Preferred C₄-C₅-*tert*-alkyl is *tert-*butyl (*t*-Bu). Preferred mono, di- or triphenyl substituted methyl is benzyl (Bn).

The protected hydroxyethyl group as a side chain signed as R in the compound of formula **A** is selected from a group of substituents described by the formula 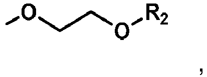 wherein R₂ is a protecting group, selected from benzyl (Bn), *tert-*butyl (t-Bu), *tert-*butyldimethylsilyl (TBDMS), methoxymethyl (MOM), acetyl (Ac) or benzoyl (Bz).

A specific embodiment of the invention is to provide a process for obtaining a compound of formula **VIII** or **IX:** 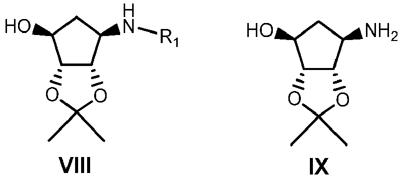 wherein R₁ is C₄-C₅-*tert*-alkyl, mono-, di-, or triphenyl substituted methyl, preferably R₁ is benzyl (Bn), by providing a compound of formula **VII** 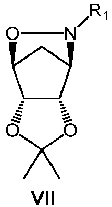 wherein R₁ is C₄-C₅-*tert*-alkyl or mono-, di- or triphenyl substituted methyl, and reducing said compound to yield the compound of formula **VIII** or **IX.** Preferably, R₁ is benzyl.

The compound of formula **VII** is prepared by a process comprising:
a) preparing the compound of formula **V** as described above,
b) treating the obtained compound of formula **V** with N-monosubstituted hydroxylamine to give a compound of formula **VI** 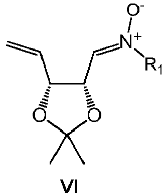 wherein R₁ is C₄-C₅-*tert*-alkyl or mono-, di- or triphenyl substituted methyl, and
c) thermally transforming the obtained compound of formula **VI** to yield the compound of formula **VII.**

Preferably, the compound of formula **V** is not isolated in step a).

Compound of formula **IX** can be further N-substituted to yield the compound of formula **VIII*** 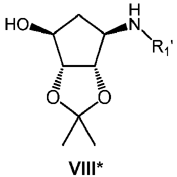 wherein R₁' is C₄-C₅-*tert*-alkyl or mono-, di- or triphenyl substituted methyl, *tert-*butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (t-Bu) or fluorenylmethoxycarbonyl (Fmoc).

Another specific embodiment of the present invention is to provide a process for obtaining a compound of formula **XI** or **XI*** or a salt thereof: 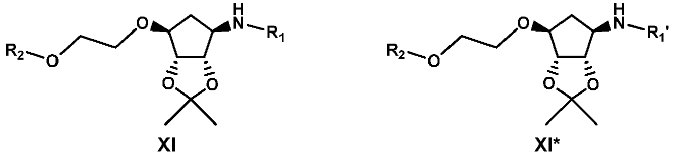 wherein R₁ is hydrogen, C₄-C₅-*tert*-alkyl or mono-, di- or triphenyl substituted methyl; R₁' is hydrogen, C₄-C₅-*tert*-alkyl or mono-, di- or triphenyl substituted methyl, *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert-*butyl (t-Bu) or fluorenylmethoxycarbonyl (Fmoc); and R₂ is hydrogen, benzyl (Bn), *tert*-butyl, *tert*-butyldimethylsilyl (TBDMS), methoxymethyl (MOM), acetyl (Ac) or benzoyl (Bz).

Said process comprises the following steps:
a) preparing the compound of formula **VIII** or **VIII*** as described above, and
b) contacting the obtained compound of formula **VIII** or **VIII*** with a compound of formula **X** 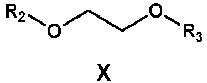 wherein R₂ is hydrogen, Bn, *t*-Bu, TBDMS, MOM, Ac or Bz, and R₃ is p-toluenesulfonyl (Ts), methanesulfonyl (Ms), Br or Cl to yield a compound of formula **XI** or **XI*,** respectively, and
c) optionally converting a compound of formula **XI** or **XI*** to a salt thereof.

Another embodiment of the present invention is to provide a process for obtaining a compound of formula **XII** or **XII*** or a salt thereof: 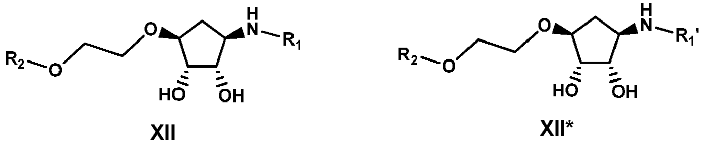 wherein R₁ is hydrogen, C₄-C₅-*tert*-alkyl or mono-, di- or triphenyl substituted methyl; R₁' is hydrogen, C₄-C₅-*tert*-alkyl or mono-, di- or triphenyl substituted methyl, *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert-*butyl (t-Bu) or fluorenylmethoxycarbonyl (Fmoc);and R₂ is hydrogen, benzyl (Bn), *tert*-butyl, *tert*-butyldimethylsilyl (TBDMS), methoxymethyl (MOM), acetyl (Ac) or benzoyl (Bz).

According to a preferred embodiment, the compound of formula **XV** or a salt thereof 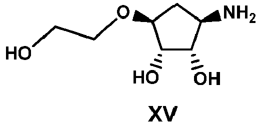 is prepared by comprising the steps of
(0-1) providing a compound of formula **XI** or **XI*,** respectively, or a salt thereof 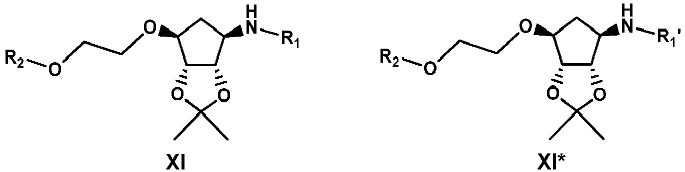 wherein R₁, R₁' and R₂ are as defined above,
(0-2) directly converting a compound of formula **XI** or **XI*** to a compound of formula **XV,** and
(0-3) optionally converting a compound of formula **XV** to a salt thereof.

Alternatively, the compound of formula **XV** or a salt thereof can be obtained by first carrying out a deprotection reaction of the cyclopentane hydroxyl group and subsequently converting the compound of formula **XII** or **XII*,** respectively to a compound of formula **XV**, by comprising the steps of:
(0-1') providing a compound of formula **XI** or **XI*,** respectively, or a salt thereof
(0-2') converting the compound of formula **XI** or **XI*,** respectively, or a salt thereof to a compound of formula **XII** or **XII*,** respectively, or a salt thereof 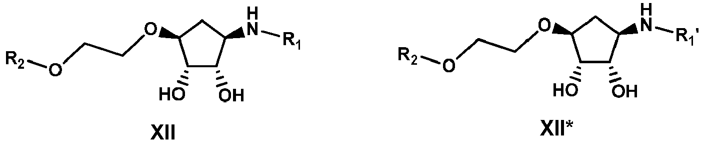 wherein R₁, R₁' and R₂ are as defined above,
(0-3') converting the compound of formula **XII** or **XII*,** respectively, or a salt thereof to a compound of formula **XV**,
(0-4') optionally converting a compound of formula **XV** to a salt thereof.

In another alternative, the compound of formula **XV** or a salt thereof can be obtained by first carrying out a deprotection reaction of the removal of groups R₁/R₁' and/or R₂ and subsequently converting partially deprotected intermediates to a compound of formula **XV,** by comprising the steps of:
(0-1 ") providing the compound of formula **XI** or **XI*** or a salt thereof
(0-2") converting the compound of formula **XI** or **XI*** to a compound of formula **XIII** 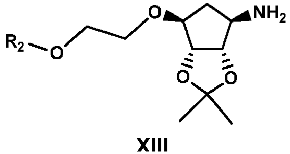 wherein R₂ is as defined above,
(0-3") converting the compound of formula **XIII** to a compound of formula XV, and
(0-4") optionally converting the compound of formula XV to a salt thereof.
   or
(0-1"') providing the compound of formula **XI** or **XI*** or a salt thereof
(0-2"') converting the compound of formula **XI** or **XI*** to a compound of formula **XIV** 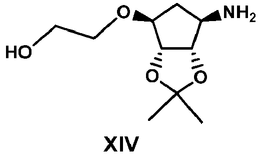
(0-3"') converting the compound of formula **XIV** to a compound of formula XV, and
(0-4"') optionally converting the compound of formula XV to a salt thereof.

A summary of the afore-mentioned ways to prepare the compound of formula XV is shown in Scheme 9.

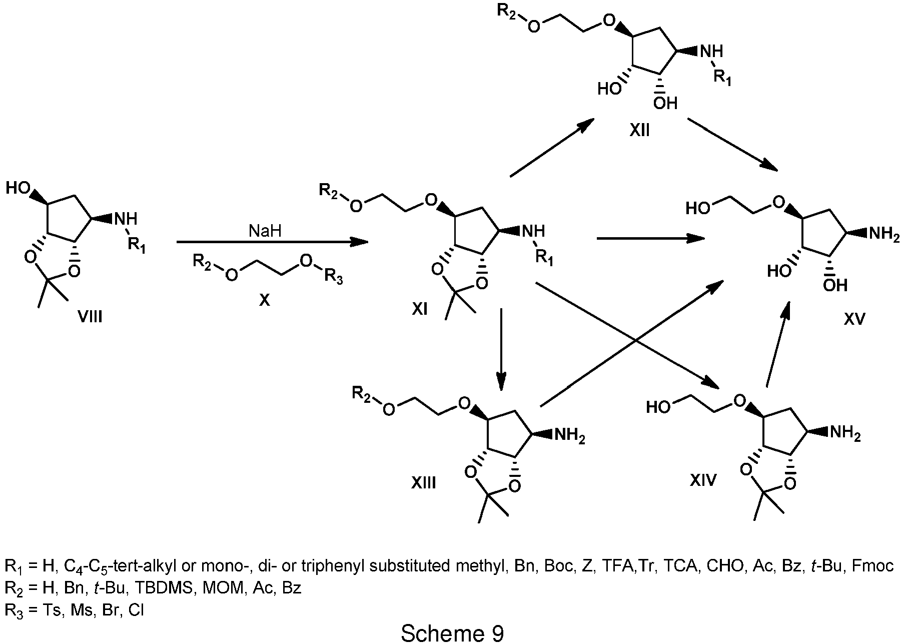

Alternatively, a compound of formula **XV** can be prepared for example by acid hydrolysis from **XIV** (Scheme 10), which can be prepared as described in WO 01/92263.

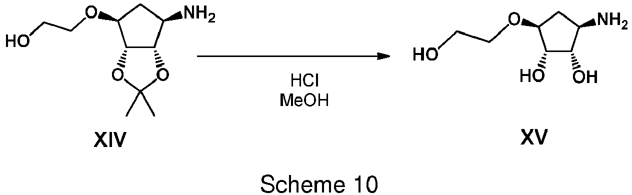

Optionally, the intermediates **XI, XI*, XII, XII*** and **XV** can be converted to a salt, thereby providing for ease of isolation/purification. A suitable salt of said intermediates is a salt of organic acid, for example an organic achiral acid such as acetic, trifluoroacetic, oxalic, maleic, fumaric or *p*-toluenesulphonic acid, or an organic chiral acid such as L-tartaric acid, dibenzoyl-L-tartaric acid or di-*p*-toluoyl-L-tartaric acid. Preferred salts of intermediates **XI, XI*, XII, XII*** and **XV** are fumarate, maleate and oxalate.

In another embodiment, the compound of formula **IV** is prepared by a process comprising
a) providing a compound of formula I 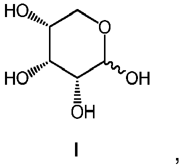
b) contacting the compound of formula **I** with acetone and methanol, or acetone ketals and methanol, in acidic medium to obtain a compound of formula **II** 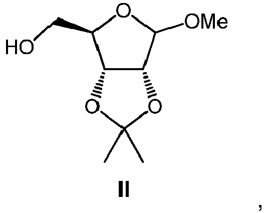
c) contacting the compound of formula **II** with chlorides or anhydrides of sulfonic acids to give a compound of formula **III** 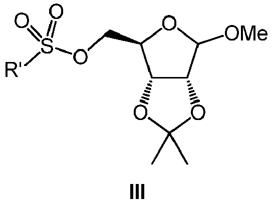 wherein R' is unsubstituted or fluoro substituted C₁-C₄-alkyl, unsubstituted or methyl, methoxy, bromo, nitro substituted phenyl, or 10-camphoryl;
d) optionally purifying the compound of formula **III** by recrystallization, and
e) converting the compound of formula **III** into the compound of formula **IV** by treatment with metal or quaternary ammonium halides.

Preferably, steps (a) to (c) are performed without isolating the compound of formula **II.**

In the first step of the process according to the invention the compound of formula **I** (D-ribose) is converted into a crystalline sulfonated compound of formula **III**

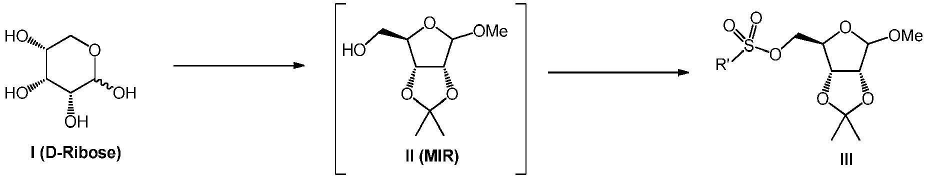

In the first part of the transformation the glycol group of ribose is protected using 2,2-dimethoxy propane and/or acetone in methanol in the presence of catalytic amounts of acid. The intermediate ((3aR,4R,6aR)-6-methoxy-2,2-dimethyltetrahydrofuro[3,4-d][1,3]dioxol-4-yl)methanol **(II)** is not isolated in a pure state, but the reaction is treated by a base, preferably selected from the base used in the next step, most preferably pyridine. Most of volatile compounds solvents are then removed, the residue is extracted by an organic solvent selected from chlorinated hydrocarbons, esters or ethers, preferably by methyl *tert*-butyl ether, the extract is optionally dried and solution is concentrated and diluted by a pyridine type solvent, selected from pyridine, picolines or lutidine, preferably by pyridine. The obtained mixture is sulfonated by an addition of a chloride or anhydride of a sulfonic acid selected from unusubstituted or fluoro substituted C₁-C₄-alkanesulfonic acids, or unsubstituted or methyl, methoxy, bromo, nitro substituted, preferably para substituted benzenesulfonic acids, or camphor-10-sulfonic acid, followed by precipitation by addition of water and isolation by filtration. The sulfonates of formula **III,** such as ((3aR,4R,6aR)-6-methoxy-2,2-dimethyltetrahydrofuro[3,4-d][1,3]dioxol-4-yl)methyl 4-methylbenzenesulfonate **(III',** compound **III,** R' = p-tolyl) or ((3aR,4R,6aR)-6-methoxy-2,2-dimethyl-tetrahydrofuro[3,4-d][1,3]dioxol-4-yl)methyl methanesulfonate **(III",** compound **III,** R' = Me) are solid crystalline materials, which are optionally purified by recrystallization from appropriate solvent such as 2-propanol to give very pure compounds.

In the further step of the invention sulfonates of Formula **III** are converted to bromo derivative of Formula **IV** by treating with corresponding metal or quaternary ammonium halides, preferably by tetrabutylammonium chloride, lithium bromide or sodium iodide.

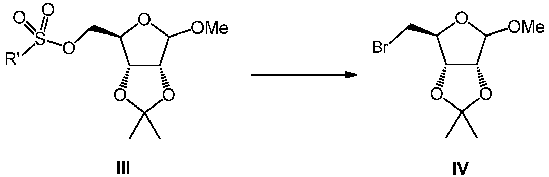

The conversion is carried out in a medium consisting of a polar solvent, selected from nitriles, amides or ketones, preferably in ketones, most preferably in butanone. All three representatives are oily materials. There is an advantage of using solid sulfonated derivatives of formula **III** in comparison to the methods, which use oily material such as the compound of formula **II.** Solid material can be recrystallized in order to enhance the purity which further leads to preparation of liquid bromo derivative of formula **IV** with enhanced purity in the next step. Such material can be used in further steps without low pressure distillation or column chromatography. Furthermore, the intermediate **IV** can be simply isolated by washing out inorganic salts with further evaporation of solvent. The solvent does not need to be completely evaporated as minor amounts of the solvent do not influence further reaction procedure.

The next technological step of the invention consists of two reaction steps. The first step includes the reduction-elimination opening of tetrahydrofuran ring to give compound **V.** The reducing agent is selected from organolithium compounds or elementary metals, preferably zinc. Using prior art procedures, such as butyl lithium in THF (Eur. J. of Org. Chem. 2001, 1293-1308) at low temperatures or zinc in refluxing alcohols (J. Org. Chem. 1995, 60, 7849-56, Tetrahedron: Asymmetry 2010, 21, 216-221), the compound of formula **IV** is converted to the compound of formula **V** in poor yields, or is practically not converted (see Example 7). We surprisingly found that *in situ* activation of zinc by acid such as hydrochloric acid or acetic acid together with addition of catalytic amounts of copper salts to activated zinc or the use of activated zinc-copper couple in place of zinc alone enhances the conversion of **IV** to **V** to acceptable yields at 20-40 °C (see Examples 8 and 9).

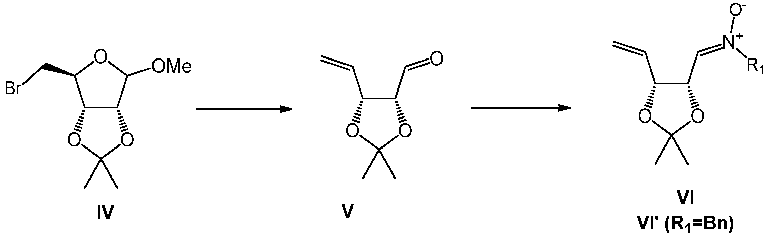

Intermediate **V** is unstable oil, so it can be further transformed without isolation with N-monosubstituted hydroxylamines into the compound of Formula **VI,** wherein R₁ is selected from C₄-C₅-*tert*-alkyl or mono-, di-, or triphenyl substituted methyl. Preferably N-(((4S,5R)-2,2-dimethyl-5-vinyl-1,3-dioxolan-4-yl)methylene)-1-phenylmethanamine oxide **(VI')** is prepared by first reacting it with *N*-benzylhydroxylamine or its salt in water miscible solvents preferably selected from C₁-C₄-alcohols, most preferably methanol and water, optionally (if salt of the *N*-benzylhydroxylamine is used) in the presence of a base, and subsequent crystallization from water. **VI'** is isolated directly from reaction mixture of **V** after removal of excess zinc by filtration and subsequent precipitation of product with water after solvent removal.

**VI'** can then be thermally transformed into (3aS,4S,7R,7aS)-6-benzyl-2,2-dimethyltetrahydro-3aH-4,7-methano[1,3]dioxolo[4,5-d][1,2]oxazine **(VII')** by heating **VI'** in appropriate solvent such as toluene, chlorobenzene or xylenes at elevated temperatures. Surprisingly, toluene as a more industry friendly solvent gives even better results than prior art chlorobenzene isolating crude **VII'** almost quantitatively.

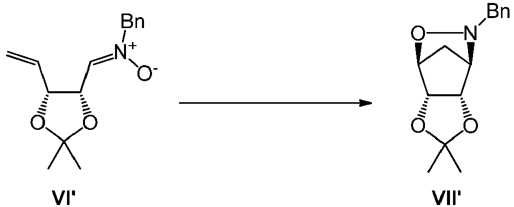

Scheme 11 shows a summary of representative steps of the process of present invention.

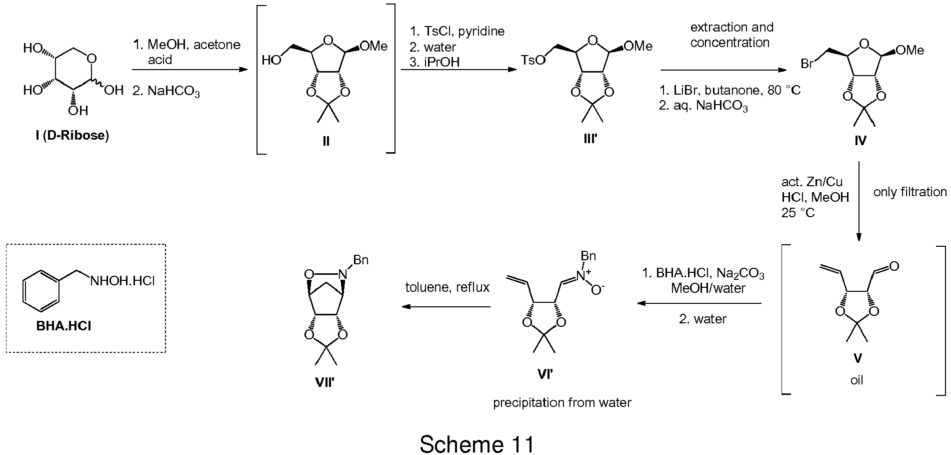

**VII'** is a solid and stable intermediate, which can be stored under standard storage conditions for several weeks, so it is suitable raw material for preparation of higher intermediates in the synthesis of P2Y12 receptor antagonists, such as **IX, XIV** and **XV.**

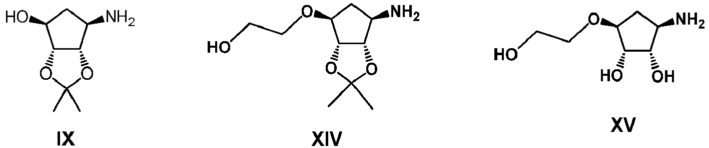

In one option intermediate **IX** can be obtained by reduction of **VII'** in the presence of hydrogen and metal catalyst such as palladium on charcoal (J. Org. Chem. 2005, 70 6884-90) or Raney nickel in a solvent such as methanol.

In another option **VII'** is only partially reduced to *N*-benzyl derivative **VIII'** by zinc in appropriate solvent (J. Chem. Soc. Perkin Trans. I., 1994, 613-14), which can be further debenzylated to **IX** by reduction with hydrogen or hydrogen donor, such as ammonium formate, and metal catalyst such as palladium on charcoal or Raney nickel and in an appropriate solvent such as methanol.

Analogues of **VIII',** represented by Formula **VIII*,** wherein R₁' is selected from hydrogen, C₄-C₅-*tert*-alkyl, mono-, di-, or triphenyl substituted methyl, *tert*-butyloxycarbonyl (Boc), benzyloxycarbonyl (Z), trifluoroacetyl (TFA), trichloroacetyl (TCA), formyl, acetyl (Ac), benzoyl (Bz), or fluorenylmethyloxycarbonyl (Fmoc), are prepared from **IX** by corresponding substituting reagents according to well-known approaches from literature or optionally from corresponding analogues of formula **VI.** Optionally some analogues, such as *t*-butyl or trityl can be prepared from corresponding compounds of formula **VII** (Scheme 12).

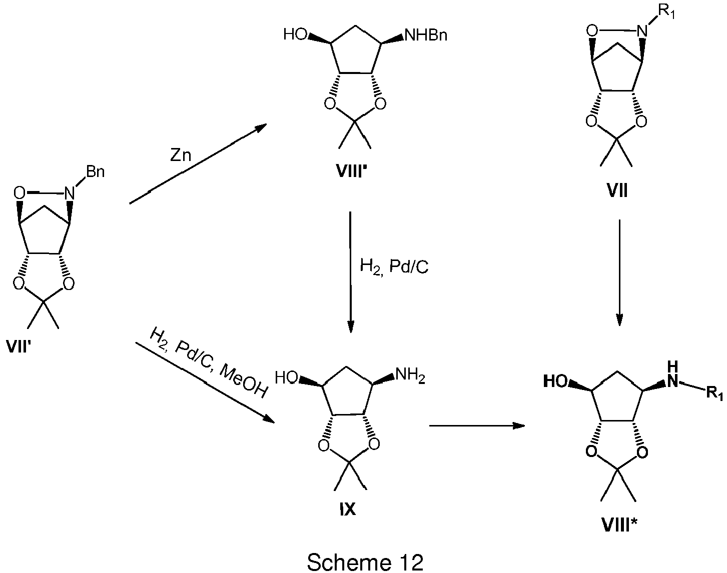

In the following the present invention will be described in further detail by illustrative, nonlimiting examples.

### Example 1: Preparation of ((3aR,4R,6aR)-6-methoxy-2,2-dimethyltetrahydrofuro[3,4-d] [1,3]dioxol-4-yl)methanol (II)

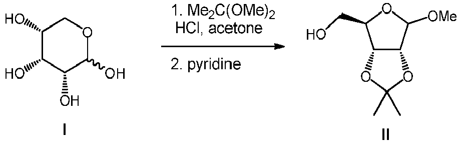

To a mixture of I (**D-ribose**) (100 g), acetone (2.0 L), 2,2-dimethoxypropane (200 mL) and methanol (300 mL) was added a saturated solution of hydrochloride in methanol (100 mL) and the resulting mixture was stirred at 25 °C for 22 hours. Pyridine (55 mL) was added and the solvent was removed under reduced pressure. The oily residue was added to a mixture of water (300 mL) and methyl *tert*-butyl ether (300 mL; MTBE). Phases were separated and the water phase was extracted two times by 300 mL of MTBE. All MTBE fractions were combined and washed twice with saturated aqueous solution of CuSO₄. Combined CuSO₄ fractions were extracted with MTBE (100 mL) and MTBE fractions were combined. Combined MTBE fractions were concentrated under reduced pressure and the product was distilled under reduced pressure (0.016 mbar, T= 90-94 °C) to yield **II** (106.4 g, 78.2 %) as colorless oil.
¹H NMR (CDCl₃): δ 1.30 (s. 3H), 1.46 (s, 3H), 3.41 (s, 3H), 3.63 (m, 2 H), 4.42 (m, 1H), 4.56 (d, 1H), 4.81 (m, 1H), 4.95 (s, 1H).

### Example 2: Preparation of ((3aR,4R,6aR)-6-methoxy-2,2-dimethyltetrahydrofuro[3,4-d] [1,3]dioxol-4-yl)methanol (II)

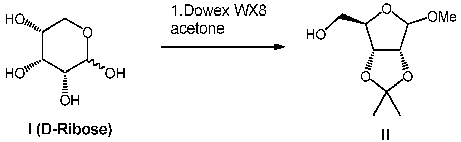

To a mixture of I **(D-ribose)** (150 g), acetone (0.75 L) and methanol (300 mL) was added dry Dowex WX8 strongly acidic resin (30 g) and the resulting mixture was stirred at 25 °C for 3 days. Dowex was filtered off (washed with 30 mL of acetone) and sodium hydrogen carbonate (15 g) was added. The resulting mixture was stirred for 10 minutes, the solvents were removed, water (0.3 L) was added and the product was extracted into methyl tert-butyl ether (4 x 0.25 L). Combined MTBE fractions were dried over magnesium sulfate, filtered and concentrated under reduced pressure to yield **II** (184 g, 90 %).

### Example 3: Preparation of ((3aR,4R,6aR)-6-methoxy-2,2-dimethyltetrahydrofuro[3,4-d] [1,3]dioxol-4-yl)methyl 4-methylbenzenesulfonate (III') from distilled II

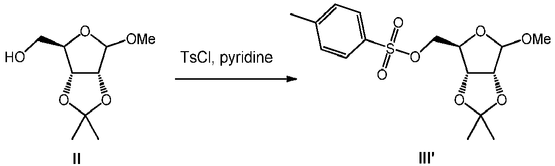

To a mixture of **II** (20.0 g) and pyridine (50 mL) was added p-toluenesulfonyl chloride (27.6 g, TsCl) at -10 °C. The resulting mixture was stirred at -10 to -5 °C for 4 hours and poured into an ice-water mixture (100 g). The precipitated solid **III'** was filtered off, washed with water (2 x 50 mL) and dried to yield **III'** (32.5 g, 93 %).

¹H NMR (CD₃OD): δ 1.27 (s, 3H), 1.41 (s, 3H), 2.47 (s, 3H), 3.20 (s, 3H), 4.01 (m, 2H), 4.24 (m, 1H), 4.52 (d, 1H), 4.60 (d, 1H), 4.89 (s, 1H), 7.47 (m, 2H), 7.82 (m, 2H) ppm.

### Example 4: Preparation of ((3aR,4R,6aR)-6-methoxy-2,2-dimethyltetrahydrofuro[3,4-d] [1,3]dioxol-4-yl)methyl methanesulfonate (III") from distilled II

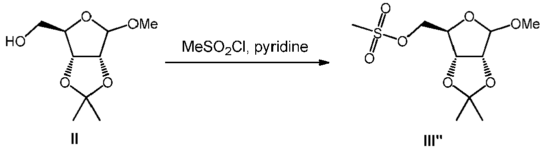

To a mixture of **II** (30.0 g, 0.15 mol) and pyridine (75 mL) was added methanesulfonyl chloride (24.9 g, TsCl) at 5 °C. The resulting mixture was stirred at 0 to 8 °C for 3 hours, then at 25 °C for 16 hours and poured into an ice-water mixture (200 g). The precipitated solid **III"** was filtered off, washed with water and dried. The resulting solid was dissolved in 2-propanol (60 mL) at elevated temperature, cooled and to the mixture was added n-hexane (50 mL). The precipitated solid was filtered off and dried to yield **III"** (28.3 g).

¹H NMR (CDCl₃): δ 1.33 (s, 3H), 1.49 (s, 3H), 3.06 (s, 3H), 3.35 (s, 3H), 4.22 (m, 2H), 4.42 (m, 1H), 4.61 (m, 1H), 4.71 (m, 1H), 5.00 (s, 1H) ppm.

### Example 5: Preparation of ((3aR,4R,6aR)-6-methoxy-2,2-dimethyltetrahydrofuro[3,4-d] [1,3]dioxol-4-yl)methyl 4-methylbenzenesulfonate (III') from I (D-ribose) without distillation

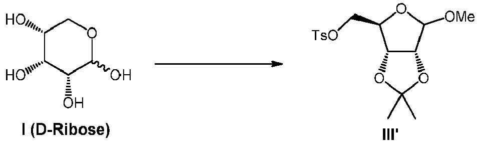

To a mixture of **I** (500 g, 3.34 mol), acetone (10.0 L), 2,2-dimethoxypropane (1.0 L) and methanol (1.4 L) was added a saturated solution of hydrochloride in methanol (0.60 L) and the resulting mixture was stirred at 25 °C for 19 hours. Pyridine (275 mL) was added and the solvents were removed under reduced pressure. The oily residue was treated by water (1.5 L) and methyl tert-butyl ether (1.5 mL). Phases were separated and the water phase was extracted two times by 1.5 L of MTBE. All MTBE fractions were combined, dried with magnesium sulfate, filtered and concentrated under reduced pressure to give crude **II** (661 g).

To a fraction of thus obtained **II** (227 g) was added pyridine (570 mL). The mixture was cooled to -10 °C and p-toluenesulfonyl chloride (314 g, TsCl) was added. The resulting mixture was stirred at 0-5 °C for 5 hours and poured into cool (18 °C) water (1.5 L). The precipitated solid **III'** was filtered off, washed with water (2 x 200 mL) and dried. The product was re-crystallized from 2-propanol (600 mL) and dried to yield pure **III'** (279 g, 68 % from **I**).

### Example 6: Preparation of (3aS,4S,6aR)-4-(bromomethyl)-6-methoxy-2,2-dimethyltetra-hydrofuro[3,4-d][1,3]dioxole (IV)

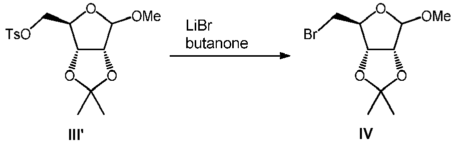

A mixture of **III'** (6.0 g), lithium bromide (3.0 g) and butanone (60 mL) was stirred at 80 °C for 3 hours and another portion of lithium bromide was added (3.5 g) and stirred at 80 °C for another 19 hours. The resulting mixture was cooled to 20-25 °C, washed 3 times with saturated aqueous sodium hydrogen carbonate (3 x 30 mL) and concentrated under reduced pressure to yield **IV** (4.98 g, containing some butanone) as oil.

¹H NMR (CDCl₃): δ 1.30 (s, 3H), 1.46 (s, 3H), 3.27-3.44 (m, 5H), 4.36 (m, 1H), 4.59 (d, 1H), 4.75 (d, 1H), 4.99 (s, 1H) ppm.

### Example 7: Preparation of (4R,5R)-2,2-dimethyl-5-vinyl-1,3-dioxolane-4-carbaldehyde (V) - Comparative example

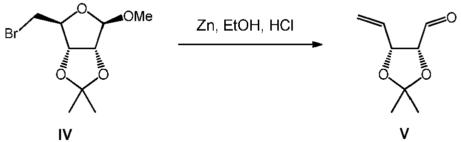

A mixture of zinc (4.1 g), ethanol (40 mL) and 4M aqueous HCl (4.0 mL) was stirred at 25 °C for 5 minutes, then **IV** (4.4 g) was added and the resulting mixture was stirred at 25 °C for 24 hours. GC analysis revealed 39 area% (solvent not integrated) of (4R,5R)-2,2-dimethyl-5-vinyl-1,3-dioxolane-4-carbaldehyde and 59 area% of unreacted starting material. After additional 6 days of stirring GC analysis revealed 54 area% (solvent not integrated) of (4R,5R)-2,2-dimethyl-5-vinyl-1,3-dioxolane-4-carbaldehyde and 19 area% of unreacted starting material.

### Example 8: Preparation of N-(((4S,5R)-2,2-dimethyl-5-vinyl-1,3-dioxolan-4-yl)methylene)-1-phenylmethanamine oxide (VI')

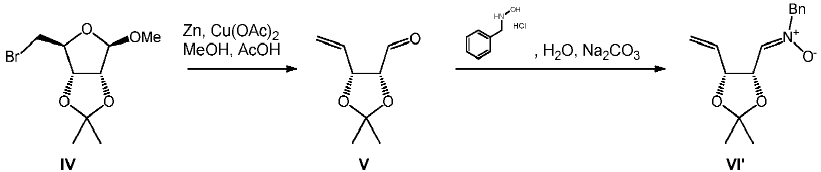

A mixture of zinc (14 g), methanol (160 mL), acetic acid (0.3 mL) and copper (**II**) acetate (1.4 g) was stirred at 45 °C for 0.5 hour, cooled to 35 °C, **IV** (6.4 g) was added and the resulting mixture was stirred at 35 °C for 4 hours and then at 25 °C for 2 days. The resulting mixture (GC analysis revealed 82 area% of **V**) was filtered through Celite and water (10 g), sodium carbonate (3 g) and *N*-benzylhydroxylamine hydrochloride (2.75 g) were added. The resulting mixture was stirred at 25 °C for 0.5 hour and methanol was removed under reduced pressure, water (200 mL) was added ant the mixture was stirred for 30 min, product was filtered off and and dried under reduced pressure to yield **VI'** (7.27 g).

### Example 9: Preparation of (4R,5R)-2,2-dimethyl-5-vinyl-1,3-dioxolane-4-carbaldehyde (V)

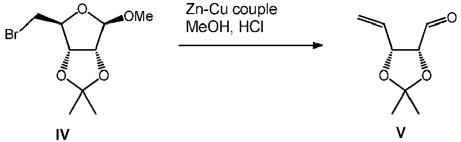

A mixture of zinc-copper couple (6.4 g), methanol (32 mL) and 4M aqueous HCl (0.64 mL) was stirred at 25 °C for 5 minutes, then **IV (BROMIR)** (3.34 g) was added and the resulting mixture was stirred at 25 °C for 18 hours. GC analysis revealed 90 area% (solvent not integrated) of (4R,5R)-2,2-dimethyl-5-vinyl-1,3-dioxolane-4-carbaldehyde **(V, ELMIR).**

### Example 10: Preparation of (3aS,4S,7R,7aS)-6-benzyl-2,2-dimethyltetrahydro-3aH-4,7-methano[1,3]dioxolo[4,5-d][1,2]oxazine (VII')

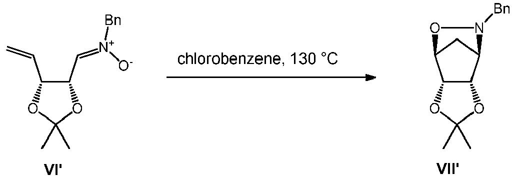

A mixture of **VI'** (9.64 g) and chlorobenzene (0.15 L) was stirred at 130 °C for 1 hour. The resulting mixture was cooled to 60 °C and chlorobenzene was removed by distillation under reduced pressure. The residue was re-crystallized from 2-propanol (70 mL) to give **VII'** (5.23 g, 54 %) as dark crystals.

¹H NMR (CDCl₃): δ 1.28 (s, 3H), 1.44 (s, 3H), 2.04 (m, 2H), 3.56 (s, 1H), 3.71 (d, 1H), 4.02 (d, 1H), 4.23 (m, 1H), 4.29 (m, 1H), 4.46 (1H), 7.25-7.36 (m, 5H) ppm.

### Example 11: Preparation of (3aS,4S,7R,7aS)-6-benzyl-2,2-dimethyltetrahydro-3aH-4,7-methano[1,3]dioxolo[4,5-d][1,2]oxazine (VII')

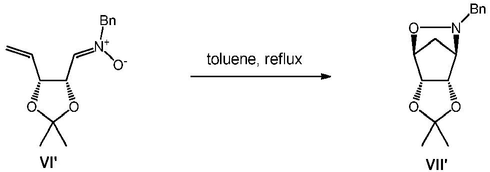

A mixture of **VI'** (35 g) and toluene (0.70 L) was stirred at reflux temperature for 5 hours. The solution was cooled to 90 °C, activated charcoal was added (5 g) and the resulting mixture was stirred at 90 °C for 10 minutes. Charcoal was filtered off and the solvent was removed under reduced pressure to give **VII'** (34.36 g, 98 %) as yellowish crystals.

### Example 12: Preparation of (3aS,4S,7R,7aS)-6-benzyl-2,2-dimethyltetrahydro-3aH-4,7-methano[1,3]dioxolo[4,5-d][1,2]oxazine (VII')

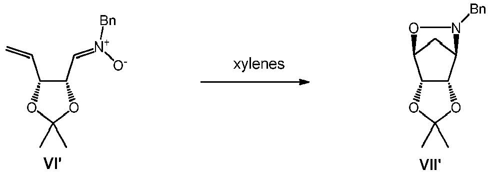

A mixture of **VI'** (16.4 g) and xylenes (0.30 L) was stirred at 130 °C for 2 hours. The solution was cooled to 25 °C, activated charcoal was added (6 g) and the resulting mixture was stirred at 25 °C for 20 minutes. Charcoal was filtered off and the solvent was removed under reduced pressure. The obtained residue was re-crystallized from 2-propanol (0.10 L) to give **VII'** (11.9 g, 73 %).

### Example 13: Preparation of (3aR,4S,6R,6aS)-6-amino-2,2-dimethyltetrahydro-3aH-cyclo-penta[d][1,3]dioxol-4-ol (IX)

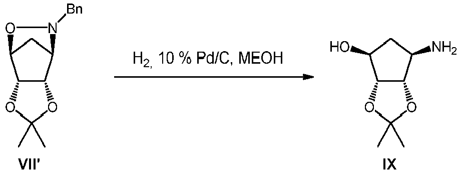

A mixture of **VII'** (60.0 g), 10 % Pd/C (6.0 g) and methanol (0.60 L) was stirred under hydrogen (3 bar) at 30 °C for 18 hours. The palladium on charcoal was removed by filtration ant the solvent was removed to give **IX** (40 g, 100 %).

¹H NMR (CDCl₃): δ 1.29 (s, 3H), 1.40 (s, 3H), 1.58 (m, 1H), 1.65 (m, 1H), 2.09 (m, 1H), 3.60 (m, 1H), 4.09 (m, 1H), 4.41 (m, 1H), 4.69 (m, 1H) ppm.

### Example 14: Preparation of (3aR,4S,6R,6aS)-6-(benzylamino)-2,2-dimethyltetrahydro-3aH-cyclopenta[d][1,3]dioxol-4-ol (VIII')

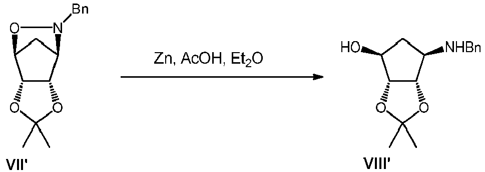

A mixture of **VII'** (20.0 g), zinc (24.9 g) and diethyl ether (1.5 L) was cooled to 0 °C and acetic acid was added slowly during 30 min while stirring. The resulting mixture was stirred at 25 °C for 3 days and filtered through Celite. To the resulting solution was added 2 M aqueous solution of NaOH so that the pH was 7.2 and the phases were separated. The upper organic phase was washed tree times with water (3 x 100 mL), dried over magnesium sulfate and the solvent was removed under reduced pressure to give **VIII'** (18.0 g, 90 %). ¹H NMR (CDCl₃): δ 1.27 (s, 3H), 1.39 (s, 3H), 1.80 (m, 1H), 2.02 (m, 1H), 3.29 (m, 1H), 3.72 (d, 1H), 3.82 (d, 1H), 4.07 (d, 1H), 4.47 (d, 1H), 4.61 (m, 1H), 7.21-7.32 (m, 5H) ppm.

### Example 15: Preparation of (3aR,4S,6R,6aS)-6-amino-2,2-dimethyltetrahydro-3aH-cyclo-penta[d][1,3]dioxol-4-ol (IX)

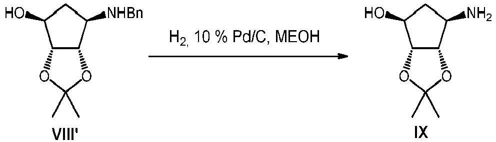

A mixture of **VIII'** (1.0 g), 10 % Pd/C (0.10 g) and methanol (10 mL) was stirred under hydrogen (3 bar) at 30 °C for 5 hours. The palladium on charcoal was removed by filtration ant the solvent was removed to give **IX** (0.59 g, 88 %).

### Example 16: Preparation of (3aS,4R,6S,6aR)-N-Benzyl-6-(2-(benzyloxy)ethoxy)-2,2-dimethyltetrahydro-3aH-cyclopenta[d][1,3]dioxol-4-amine (XI')

### a)

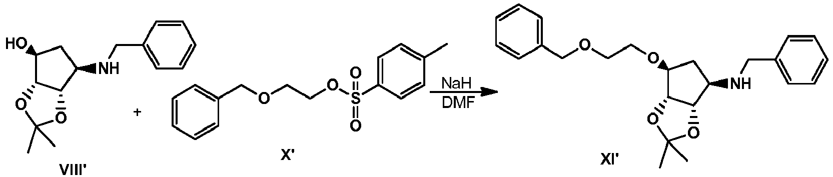

A solution of (3aR,4S,6R,6aS)-6-(benzylamino)-2,2-dimethyltetrahydro-3aH-cyclopenta-[d][1,3]- dioxol-4-ol **(VIII')** (1.0 g, 3.8 mmol) in dry DMF (10 mL) under nitrogen atmosphere was cooled at 0 °C followed by addition of NaH (60 %, 182 mg, 4.6 mmol). After stirring for 30 min at 0 °C, 2-(benzyloxy)ethyl 4-methylbenzenesulfonate (**X'**) (1.2 g, 3.8 mmol) was added and the reaction mixture was allowed to warm at room temperature. After stirring for 4 hours, the reaction mixture was quenched with water (10 mL). To the mixture was extracted 3 x 10 mL of n-hexane. The combined organic phases were dried over MgSO₄ filtered and evaporated to the dryness.

### b)

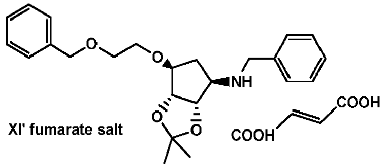

Compound **XI'** was isolated from the reaction mixture by salt formation with fumaric acid. The solution of reaction mixture of **XI'** (contained about 80 % of **XI')** in 2-butanone was warmed to 50 °C. 1 eq of fumaric acid (calculated to amount of **XI')** was added and reaction mixture was stirred at 50 °C until fumaric acid dissolution. The reaction mixture was allowed to cool at room temperature followed by addition of n-hexane. After overnight stirring at room temperature, the precipitate white salt of **XI'** was sucked off, washed with n-hexane and dried under reduce pressure at 40 °C.

### c)

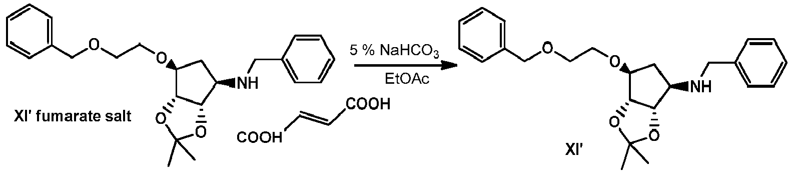

The fumarate salt of **XI'** was suspended in EtOAc and 5% aqueous solution of NaHCO₃ was added to the suspension. The mixture was stirred vigorously at room temperature for an hour. The two clear phases were separated and organic phase was washed with water, dried over MgSO₄ and evaporated to the dryness to provide pure **XI'.**

¹H NMR (CDCl₃) δ = 1.30 (s, 3H), 1.40 (s, 3H), 1.90 (d, 1H), 2.15 (m, 1H), 3.17 (m, 1H), 3.60 (m, 2H), 3.68 (m, 2H), 3.80-3.90 (m, 3H), 4.51 (s, 2H), 4.63 (m, 2H), 7.23-7.36 (m, 10H) ppm. ¹³C NMR (CDCl₃) δ = 24.0, 26.4, 33.9, 51.6, 63.0, 68.5, 69.2, 73.1, 83.9, 84.6, 84.8 110.4, 126.7, 127.5, 127.53, 128.0, 128.2, 128.3, 138.1, 140.3 ppm.

### Example 17: Preparation of 2-(((3aR,4S,6R,6aS)-6-amino-2,2-dimethyltetrahydro-3aH-cyclopenta[d][1,3]dioxol-4-yl)oxy)ethanol (XIV)

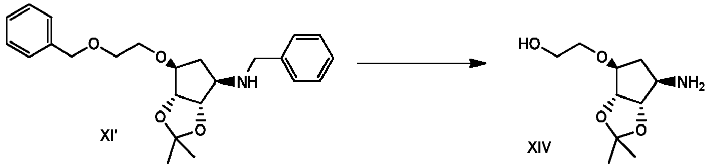

A mixture of (3aS,4R,6S,6aR)-N-benzyl-6-(2-(benzyloxy)ethoxy)-2,2-dimethyltetrahydro-3aH-cyclopenta[d][1,3]dioxol-4-amine **(XI',** 56 g), methanol (2.2 L) and 10 % Pd/C (15 g) under 10 bar of hydrogen is stirred at 40 °C for 7 days, the Pd/C is removed by filtration and methanol is removed under reduced pressure to give 19.32 g (63 %) of 2-(((3aR,4S,6R,6aS)-6-amino-2,2-dimethyltetrahydro-3aH-cyclopenta[d][1,3]dioxol-4-yl)oxy)ethanol **(XIV).**

### Example 18: Preparation of (1S,2S,3R,5S)-3-(Benzylamino)-5-(2-(benzyloxy)ethoxy)cyclopentane-1,2-diol (XII')

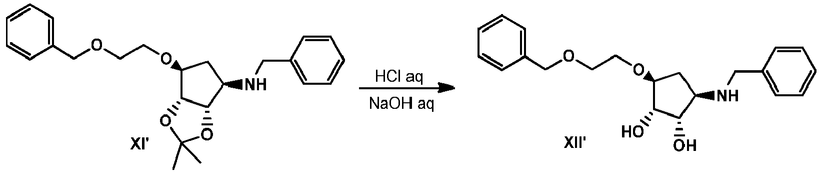

**XI'** (2.0 g, 5 mmol) was treated with 1 M HCl (20 mL) at room temperature for 7 hours followed by addition of 1 M NaOH (25 mL). The reaction mixture was extracted with 30 mL of EtOAc. Organic phase was washed with water, dried over MgSO₄ and evaporated to the dryness to provide **XII'.**

¹H NMR (CDCl₃) δ = 1.34 (m, 1H), 2.44 (m, 1H), 3.02 (m, 1H), 3.60-3.87 (m, 8H), 4.0 (m, 1H), 4.56 (s, 2H), 7.27-7.37 (m, 10H) ppm.

¹³C NMR (CDCl₃) δ = 34.6, 52.3, 61.3, 69.3, 69.8, 73.3, 75.5, 76.2, 83.8, 127.1, 127.8, 128.2, 128.5, 128.6, 137.9, 140.0 ppm.

### Example 19: Preparation of (1S,2S,3R,5S)-3-Amino-5-(2-hydroxyethoxy)cyclopentane-1,2-diol (XV)

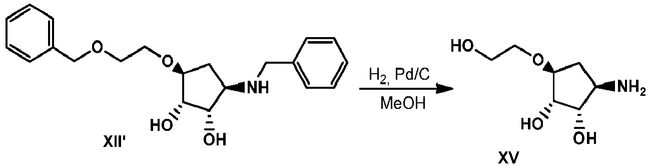

The solution of **XII'** (1.1 g, 2.8 mmol) in MeOH (15 mL) was hydrogenated at 10 bar of hydrogen for 16 hours in the presence of Pd/C (10 %, 0.2 g). The reaction mixture was passed through the pad of celite and evaporated to the dryness to provide **XV** .

¹H NMR (DMSO-d₆) δ = 1.07 (m, 1H), 2.19 (m, 1H), 2.90 (m, 1H), 3.36-3.60 (m, 6H), 3.74 (m, 1H) ppm.

¹³C NMR (DMSO-d6) δ = 36.4, 55.0, 60.4, 70.7, 75.1, 78.7, 83.4 ppm.

## Claims

1. A process for preparation of a compound of formula V 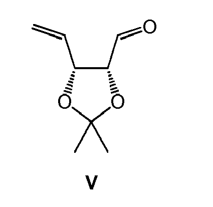 comprising:
a) providing a compound of formula **IV** 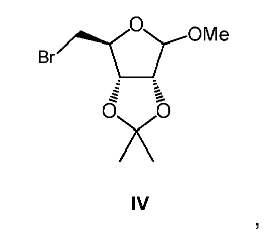 and
b) reducing the compound of formula **IV** with activated zinc in the presence of copper to give the compound of formula **V**.

2. The process according to claim 1, wherein the compound of formula **IV** is prepared by a process comprising:
a) providing a compound of formula I 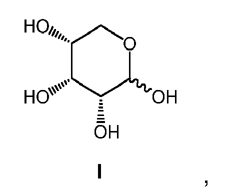
b) contacting the compound of formula **I** with acetone and methanol, or acetone ketals and methanol, in acidic medium to obtain a compound of formula **II** 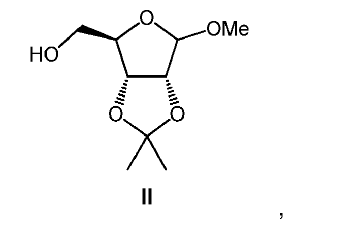
c) contacting the compound of formula **II** with chlorides or anhydrides of sulfonic acids to give a compound of formula **III** 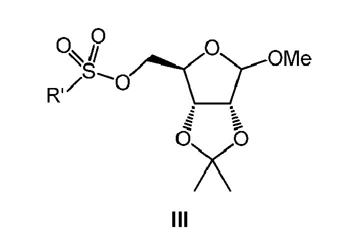 wherein R' is unsubstituted or fluoro substituted C₁-C₄-alkyl, unsubstituted or methyl, methoxy, bromo, nitro substituted phenyl, or 10-camphoryl;
d) optionaly purifying the compound of formula **III** by recrystallization, and
e) converting the compound of formula **III** into the compound of formula **IV.**

3. The process according to claim 2, wherein steps (a) to (c) are performed without isolating the compound of formula **II.**

4. A process for preparation of a compound of formula **VII** 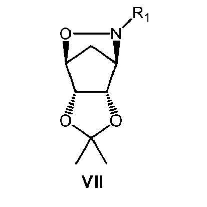 wherein R₁ is C₄-C₅-*tert*-alkyl or mono-, di- or triphenyl substituted methyl,
the process comprising:
a) preparing the compound of formula **V** according to claim 1,
b) treating the obtained compound of formula **V** with N-monosubstituted hydroxylamine to give a compound of formula **VI** 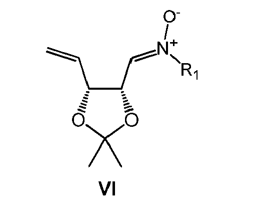 wherein R₁ is C₄-C₅-*tert*-alkyl or mono-, di- or triphenyl substituted methyl, and
c) thermally transforming the obtained compound of formula VI to yield the compound of formula VII.

5. The process according to claim 4, wherein in step (a) the compound of formula V is not isolated.

6. The process according to claim 4 or claim 5, further comprising reducing the compound of formula **VII** to yield a compound of formula **VIII** 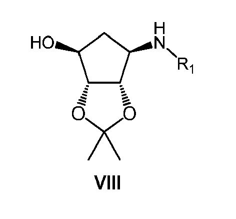 wherein R₁ is C₄-C₅-*tert*-alkyl or mono-, di- or triphenyl substituted methyl.

7. The process according to claim 4 or claim 5, further comprising transforming the compound of formula **VII** to yield a compound of formula **IX**

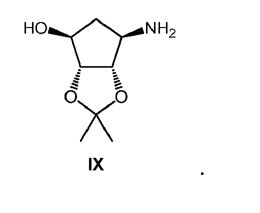

8. The process according to claim 7, further comprising N-substituting the compound of formula **IX** to yield a compound of formula **VIII*** 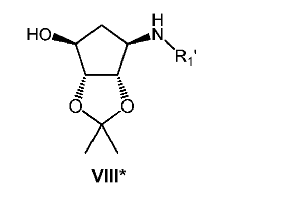 wherein R₁' is C₄-C₅-*tert*-alkyl or mono-, di- or triphenyl substituted methyl, *tert-*butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert-*butyl (t-Bu) or fluorenylmethoxycarbonyl (Fmoc).

9. A process for preparation of a compound of formula **XI** or **XI*** or a salt thereof 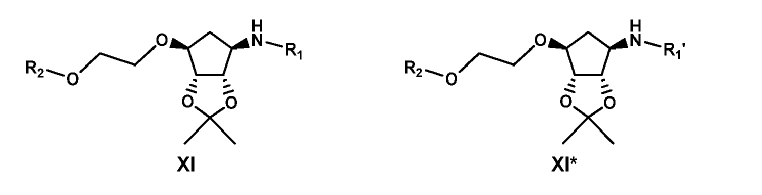 wherein R₁ is hydrogen, C₄-C₅-*tert*-alkyl or mono-, di- or triphenyl substituted methyl; R₁' is hydrogen, C₄-C₅-*tert*-alkyl or mono-, di- or triphenyl substituted methyl, *tert-*butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert-*butyl (t-Bu) or fluorenylmethoxycarbonyl (Fmoc); and R₂ is hydrogen, Bn, *t*-Bu, TBDMS, MOM, Ac or Bz,
the process comprising:
a) preparing the compound of formula **VIII** or **VIII*** according to claim 6 or 8, respectively, and
b) contacting the obtained compound of formula **VIII** or **VIII*** with a compound of formula **X** 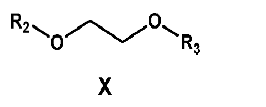 wherein R₂ is hydrogen, Bn, *t*-Bu, TBDMS, MOM, Ac or Bz, and R₃ is p-toluenesulfonyl (Ts), methanesulfonyl (Ms), Br or Cl to yield a compound of formula **XI** or **XI*,** respectively, and
c) optionally converting a compound of formula **XI** or **XI*** to a salt thereof.

10. The process according to claim 9, wherein R₁ and/or R₁' and/or R₂ is hydrogen.

11. A process for preparation of a compound of formula XV or a salt thereof 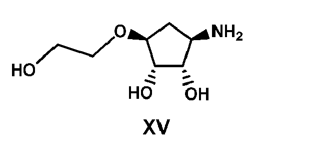 comprising the steps of
a) preparing the compound of formula **XI** or **XI*** or a salt thereof according to claim 9,
b) directly converting the compound of formula **XI** or **XI*** to a compound of formula XV, and
c) optionally converting a compound of formula XV to a salt thereof;

12. A process for preparation of a compound of formula **XV** or a salt thereof 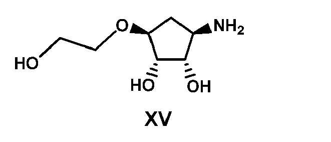 comprising the steps of
a) preparing the compound of formula **XI** or **XI*** or a salt thereof according to claim 9,
b) converting the compound of formula **XI** or **XI*** to a compound of formula **XIII** 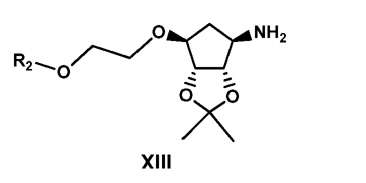 wherein R₂ is hydrogen, Bn, *t*-Bu, TBDMS, MOM, Ac or Bz,
c) converting the compound of formula **XIII** to a compound of formula **XV,** and
d) optionally converting the compound of formula **XV** to a salt thereof.

13. A process for preparation of a compound of formula **XV** or a salt thereof 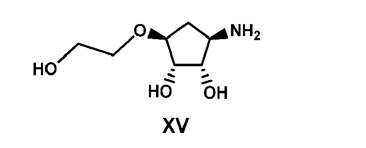 comprising the steps of
a) preparing the compound of formula **XI** or **XI*** or a salt thereof according to claim 9,
b) converting the compound of formula **XI** or **XI*** to a compound of formula **XIV** 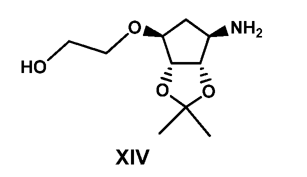
c) converting the compound of formula **XIV** to a compound of formula **XV,** and
d) optionally converting the compound of formula **XV** to a salt thereof.

14. A process for preparation of a compound of formula **XV** or a salt thereof 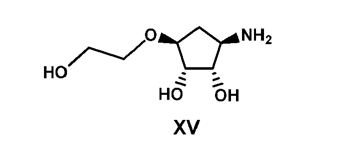 comprising the steps of
a) preparing the compound of formula **XI** or **XI*** or a salt thereof according to claim 9,
b) converting a compound of formula **XI** or **XI*** to a compound of formula **XII** or **XII*,** respectively 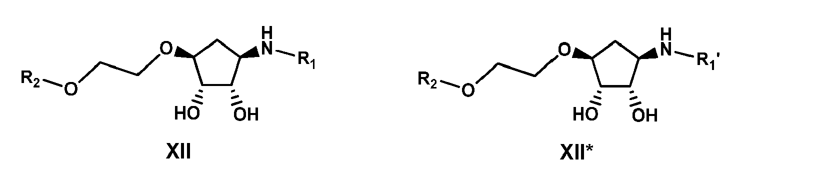 wherein R₁ is hydrogen, C₄-C₅-*tert*-alkyl or mono-, di- or triphenyl substituted methyl; R₁' is hydrogen, C₄-C₅-*tert*-alkyl or mono-, di- or triphenyl substituted methyl, *tert*-butoxycarbonyl (Boc), benzyloxycarbonyl (Cbz), trifluoroacetyl (TFA), trityl (Tr), trichloroacetyl (TCA), formyl (CHO), acetyl (Ac), benzoyl (Bz), *tert*-butyl (t-Bu) or fluorenylmethoxycarbonyl (Fmoc);and R₂ is hydrogen, Bn, *t*-Bu, TBDMS, MOM, Ac or Bz,
c) converting the compound of formula **XII** or **XII*** to a compound of formula **XV**, and
d) optionally converting the compound of formula **XV** to a salt thereof.

15. Use of the process according to any of previous claims for the synthesis of ticagrelor.
